# EUROPEAN PATENT SPECIFICATION

(11) **EP 4 101 408 B1**
(45) Date of publication and mention of the grant of the patent: **26.03.2025**
(21) Application number: 21754227.3
(22) Date of filing: 12.01.2021
(51) Int. Cl.: A61B 34/20, A61B 34/30, A61B 90/50, A61B 46/10, A61B 90/00, A61B 17/34

(54) **ROBOT FOR SURGERY**
ROBOTER FÜR CHIRURGIE
ROBOT POUR CHIRURGIE

(30) Priority: 12.02.2020 JP 2020021632
(43) Date of publication of application: 14.12.2022
(73) Proprietor: RIVERFIELD Inc., 1070052 Tokyo (JP)
(72) Inventor: KANAZAWA, Masao, Tokyo 107-0052 (JP); TANAKA, Yasushi, Tokyo 107-0052 (JP)
(74) Representative: adares Patent- und Rechtsanwälte Reininger & Partner GmbB
(86) International application number: PCT/JP2021/000706
(87) International publication number: WO 2021/161702

(56) References cited:
- WO-A1-2015/142824
- JP-A- 2001 204 738
- JP-A- 2014 148 037
- JP-A- 2017 512 547
- JP-A- 2018 019 993
- KR-A- 20110 004 496
- US-A1- 2014 195 052
- US-A1- 2015 359 597
- US-A1- 2019 076 202
- US-A1- 2019 076 202
- US-A1- 2019 209 241

## Description

### TECHNICAL FIELD

The present disclosure relates to a surgical robot for use in endoscopic surgery.

### BACKGROUND ART

Endoscopic surgery such as laparoscopic surgery is performed by the following procedure.

Specifically, an operator such as a doctor makes two or more small holes in a subject, and inserts a cylindrical trocar into each of the holes. Trocar is also called trocar.

Next, the operator inserts an endoscope, forceps, an electric scalpel or the like to each trocar, and performs surgery while looking at an image captured by the endoscope. Forceps are an instrument for gripping and pulling an internal organ or the like, and can be remotely controlled. Hereinafter, an instrument, such as an endoscope, forceps and an electric scalpel, for use in endoscopic surgery is referred to as a surgical instrument.

Patent document 2 describes a surgical robot and a setting method to reduce robot setting time by memorizing the position of a robot arm according to the kind of an operation. The surgical robot comprises a robot arm which is driven according to user manipulation, a data storage module storing setting location information of the robot arm, an information output unit outputting the actual location and setting location information of the robot arm.

### PRIOR ART DOCUMENTS

### PATENT DOCUMENTS

Patent Document 1: Japanese Patent No. 4999012
Patent Documnet 2 : KR 2011 0004496 A1

### SUMMARY OF THE INVENTION

### PROBLEMS TO BE SOLVED BY THE INVENTION

A surgical instrument such as an endoscope and forceps is inserted into a body of the subject through an incision position, that is, a site where a trocar is to be inserted. Thus, it is necessary for the surgical robot to move the surgical instrument so that a portion of the surgical instrument corresponding to the incision site is immovable.

In view of the above, the present disclosure discloses an example of a surgical robot that allows an operator to confirm "whether the surgical instrument is moving so that the portion of the surgical instrument corresponding to the incision site is immovable".

### MEANS FOR SOLVING THE PROBLEMS

The invention is defined and the problems are solved by a surgical robot according to claim 1. It is desirable that a surgical robot for use in endoscopic surgery comprises at least one of the following components, for example.

Specifically, the components are: an arm device that holds a surgical instrument used in endoscopic surgery; a drive device that drives the arm device; and a display section for displaying information; and a display processor that displays a relative positional relationship between a site where a trocar is to be inserted and a distal end position of the surgical instrument during surgery on the display section.

This allows an operator to confirm "whether the surgical instrument is moving so that a portion of the surgical instrument corresponding to the incision site is immovable. Specifically, the operator can easily and reliably recognize whether the surgical robot "recognizes the site where the trocar is to be inserted as an immovable point", in other words, whether surgery by the surgical robot is ready to be performed.

The surgical robot is configured as follows.

Specifically, an immovable point setter is provided which recognizes a position of the "site where the trocar is to be inserted" and stores the recognized position, and the display processor uses the position stored by the immovable point setter as the "site where the trocar is to be inserted". This allows an operator to easily and reliably recognize whether the immovable point setter stores the incision position as the immovable point.

It is desirable that a drape detector and a second display processor are provided. The drape detector detects whether a drape that covers the arm device is attached to the arm device. The second display processor displays a detection result by the drape detector on the display section. This allows the operator to easily and reliably recognize whether surgery by the surgical robot is ready to be performed.

### BRIEF DESCRIPTION OF THE DRAWINGS

FIG. 1 is an external view of a surgical robot according to a first embodiment.
FIG. 2 is a block diagram of the surgical robot according to the first embodiment.
FIGS. 3A to 3C are diagrams each showing a display example of a first display processor.
FIGS. 4A and 4B are diagrams each showing a display example of the first display processor.
FIG. 5 is a flowchart showing a control of an immovable point setting mode of the surgical robot according to the first embodiment.

### EXPLANATION OF REFERENCE NUMERALS

1...surgical robot, 3...robot arm, 5...control device, 7...surgical instrument, 9...arm drive device, 11...immovable point setter, 13...drive controller, 15...trocar, 17A...setting button, 17B...free displacement enabling button, 19...display section, 20...drape, 21A... first display processor, 21B... second display processor, 23...drape detector.

### MODE FOR CARRYING OUT THE INVENTION

An example embodiment of the present disclosure will be described hereinafter.

Arrows indicating directions, hatched lines, etc. shown in the drawings are provided for easy understanding of relationships between the drawings, shapes of members or portions, and others. Accordingly, a configuration of the present disclosure is not limited by the directions shown in the drawings. The drawings with hatched lines do not necessarily show cross-sectional views.

For at least a member or portion described with a reference numeral affixed thereto, there is at least one in number unless specified as "one" or the like. In other words, the member or portion may be two or more in number unless specified as "one". The surgical robot shown in the present disclosure comprises at least components such as members or portions described with reference numerals affixed thereto, and structural portions shown in the drawings.

### (First Embodiment)

### <1. Configuration of surgical robot>

The present embodiment is an example of a surgical robot for use in endoscopic surgery.

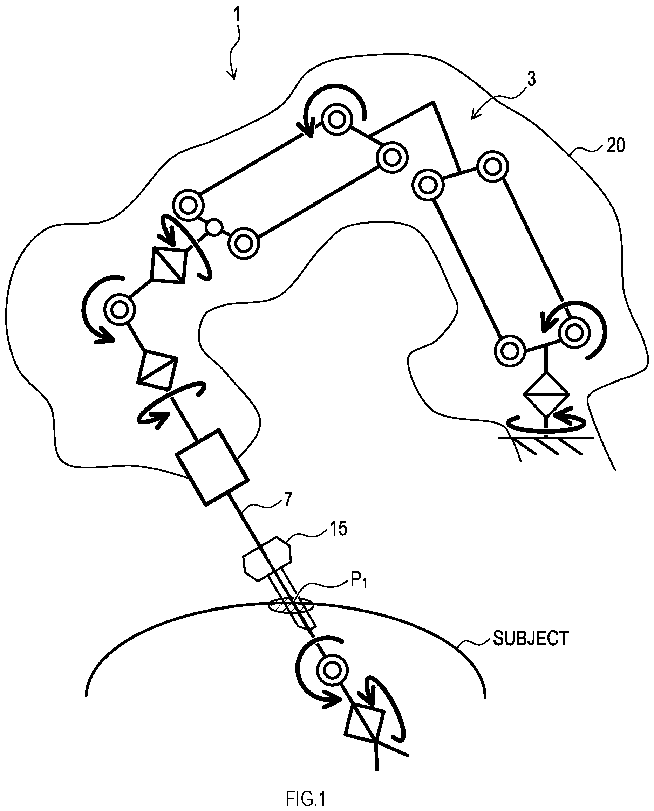

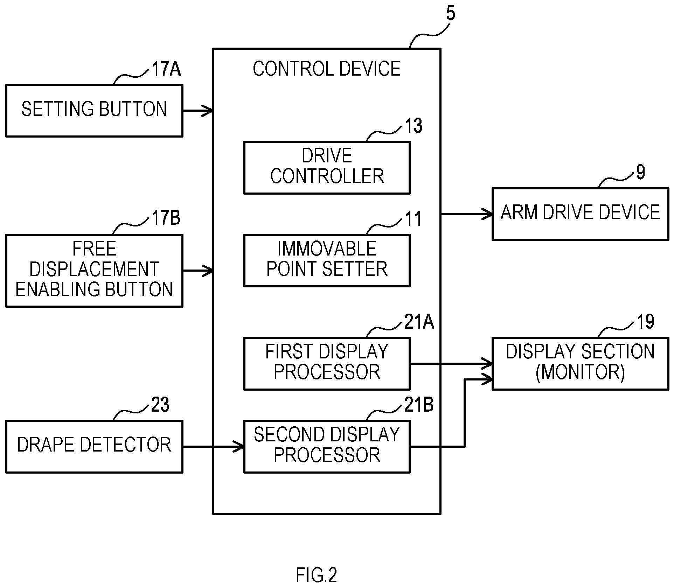

As shown in FIG. 2, a surgical robot 1 comprises a control device 5, an arm drive device 9, and a display section 19, in addition to a robot arm 3 (see FIG. 1).

### <Robot arm>

The robot arm 3 is an example of an arm device holding a surgical instrument 7, as shown in FIG. 1. Specifically, the robot arm 3 is configured by a link mechanism that has two or more joints and that can change a position of pivot.

The pivot is a position which is an immovable point when the robot arm 3 operates, regardless of a state of the robot arm 3. The surgical instrument 7 is an instrument, such as an endoscope, forceps and an electric scalpel, for use in endoscopic surgery.

The surgical instrument 7 shown in FIG. 1 is forceps. At a distal end of the forceps, a hand part for gripping and pulling an internal organ or the like is provided. The robot arm 3 is covered by a tubular drape 20. The drape 20 is a flexible, non-woven fabric covering member.

### < Arm drive device (see FIG. 2)>

The arm drive device 9 is an example of a drive device that drives the robot arm 3. The arm drive device 9 according to the present embodiment comprises two or more electric motors, an air pressure cylinder, and a pressure generator.

Each electric motor drives the corresponding joint. The air pressure cylinder applies tension to a wire that drives the surgical instrument 7 (for example, hand part of the forceps). The pressure generator supplies a compressed air to the air pressure cylinder.

### <Control device>

The control device 5 comprises an immovable point setter 11, a drive controller 13, a first display processor 21A, and a second display processor 21B.

The immovable point setter 11 recognizes a position of a site where a trocar 15 (see FIG. 1) is inserted during surgery (hereinafter, also referred to as an incision position), and stores the recognized position as a pivot P₁.

Hereinafter, a series of operations from recognition of the incision position to storage of the position, etc. by the immovable point setter 11 is referred to as immovable point setting. A state in which the immovable point setting can be performed is referred to as an immovable point setting mode.

The trocar 15 is a cylindrical member to be inserted into a hole incised in a subject. In other words, a surgical instrument 7 such as forceps and an endoscope is inserted into a body of the subject through the trocar 15 inserted to an incision site.

The drive controller 13 uses the position of the pivot P₁ to control operation of the arm drive device 9. Specifically, the drive controller 13 receives a command signal outputted from a master-side input operation device, and activates the arm drive device 9 according to the command signal.

At this time, the drive controller 13 activates the arm drive device 9 so that a portion of the surgical instrument 7 corresponding to the pivot P₁ is immovable. The mater-side input operation device is an example of an input device which is directly operated by an operator such as a doctor.

The first display processor 21A and the second display processor 21B display information on the display section 19. The display section 19 is a monitor that transmits information such as text information and image information to the user. An image captured by the endoscope is displayed on a monitor separate from the display section 19.

The first display processor 21A displays a relative positional relationship between the incision position, that is, the pivot P₁ and the distal end position of the surgical instrument 7 on the display section 19. The first display processor 21A according to the present embodiment uses image information such as figures (for example, icons) to display the relative positional relationship on the display section 19. Each icon has a figure which represents the pivot P₁ or the distal end position of the surgical instrument 7.

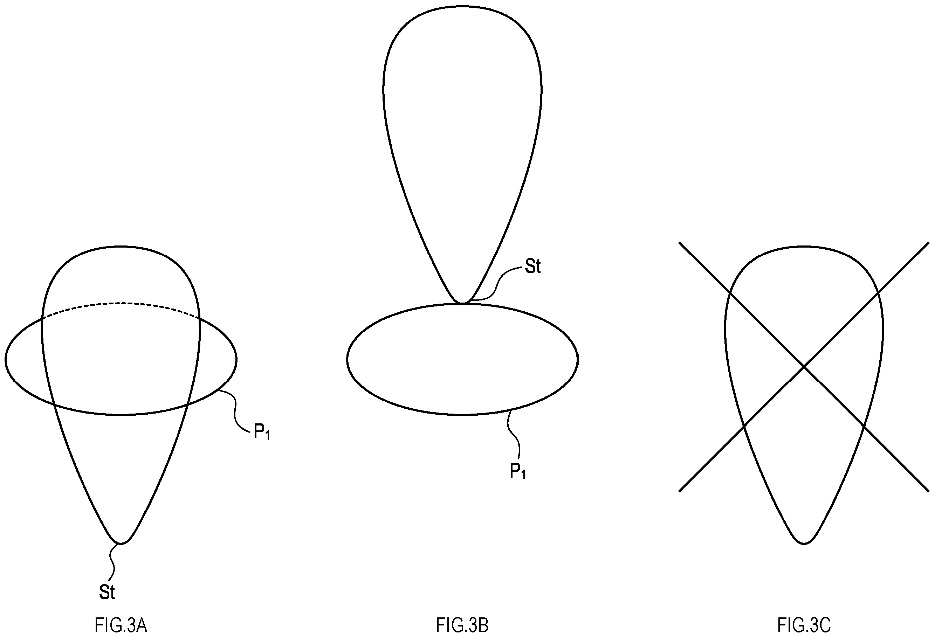

Specifically, for example, FIG. 3A shows a case where the distal end position St of the surgical instrument 7 is located inside a body relative to the pivot P₁. FIG. 3B shows a case where the distal end position St of the surgical instrument 7 is located outside the body relative to the pivot P₁. FIG. 3C shows a state in which the immovable point setting is not yet performed.

The second display processor 21B displays detection a result of the drape detector 23 (see FIG. 2) on the display section 19. The drape detector 23 detects whether the drape 20 is attached to the robot arm 3. The drape detector 23 is provided in the robot arm 3.

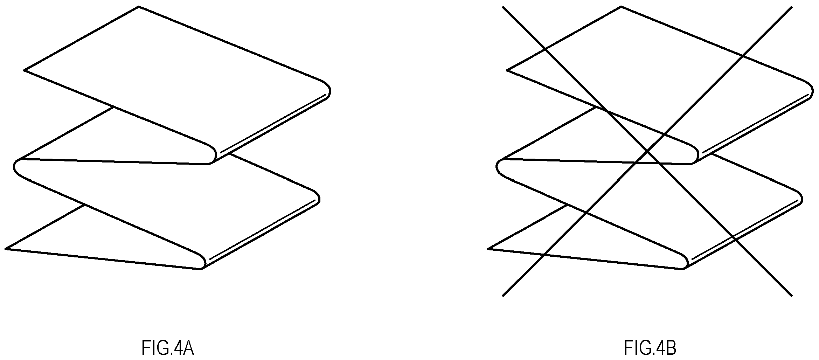

If the drape 20 is attached, the second display processor 21B displays that information (for example, see FIG. 4A) on the display section 19. If the drape 20 is not attached, the second display processor 21B displays that information (for example, see FIG. 4B) on the display section 19.

### <2. Detail of immovable point setter>

The immovable point setter 11 according to the present embodiment can execute a position recognition function and a memory function. The immovable point setter 11 uses the position recognition function and the memory function to store the position of the pivot P₁ as an immovable point.

The position recognition function is a function to recognize a distal end position of the surgical instrument 7 held by the robot arm 3. The memory function stores the distal end position recognized by the position recognition function as the pivot P₁. The pivot P₁ stored by the memory function may be, for example, a position recognized by the position recognition function. Also, the position recognized by the position recognition function is not limited to the distal end position of the surgical instrument 7. The position recognized by the position recognition function may be, for example, the incision position which is the position of a site where the trocar 15 is to be inserted during surgery.

The position recognition function according to the present embodiment recognizes the distal end position of the surgical instrument 7 by obtaining or calculating a coordinate or the like which indicates the distal end position of the surgical instrument 7 from an attitude of the robot arm 3. The memory function stores the coordinate as the pivot P₁.

To perform the immovable point setting, a surgical instrument equivalent may be used instead of the surgical instrument 7. The surgical instrument equivalent is a member having a shape similar to that of the surgical instrument 7. Specifically, for example, a rod-shaped or pipe-shaped member corresponds to the surgical instrument equivalent.

The position recognition function and memory function according to the present embodiment are implemented by a software, programs that make up the software, and a microcomputer. The microcomputer comprises a CPU, a ROM, a RAM, etc. to run the software. The software is stored in a non-volatile storage section in advance.

The surgical robot 1 has a setting button 17A, a free displacement enabling button 17B and the like, as shown in FIG. 2. The setting button 17A and the free displacement enabling button 17B are provided in at least one of the robot arm 3 and the control device 5. The robot arm 3 corresponds to an example of a slave device, and the control device 5 corresponds to an example of a master-side device.

The setting button 17A is an example of a setting operating section operated by a user. The user is one who performs an immovable point setting work. Specifically, the user is an operator or those who assist surgery. When the setting button 17A is operated, an immovable point setting mode starts or ends.

In other words, if the setting button 17A is operated in a mode other than the immovable point setting mode, the immovable point setting mode is started. If the setting button 17A is operated in the immovable point setting mode, the immovable point setting mode ends.

Specifically, if the setting button 17A is depressed for more than a specified time (for example, three seconds), the immovable point setting mode is started. When the immovable point setting mode is started, the position recognition function is enabled.

When the setting button 17A is depressed less than the specified time (for example, two seconds), the position recognition function is executed and then the memory function is executed. Thereafter, the pivot P₁ is stored as the immovable point, and the immovable point setting mode ends.

The free displacement enabling button 17B is an example of the operating section operated by the user. When the free displacement enabling button 17B is operated, the arm drive device 9 is brought into a free displacement mode. The free displacement mode is a mode in which the robot arm 3 is freely displaceable in accordance with an external force acting on the robot arm 3.

Therefore, in the free displacement mode, the user can freely displace the robot arm 3 by pushing and pulling the robot arm 3. In other words, in the free displacement mode, the user can align the distal end of the surgical instrument 7 with the incision position by pushing and pulling the robot arm 3 without operating the master-side input operation device.

The free displacement mode ends "if the free displacement enabling button 17B is operated in the free displacement mode", or "when the immovable point setting mode ends". In a state in which the free displacement mode is not started, the robot arm 3 is not displaced even if an external force acts on the robot arm 3.

### <Control in immovable point setting mode>

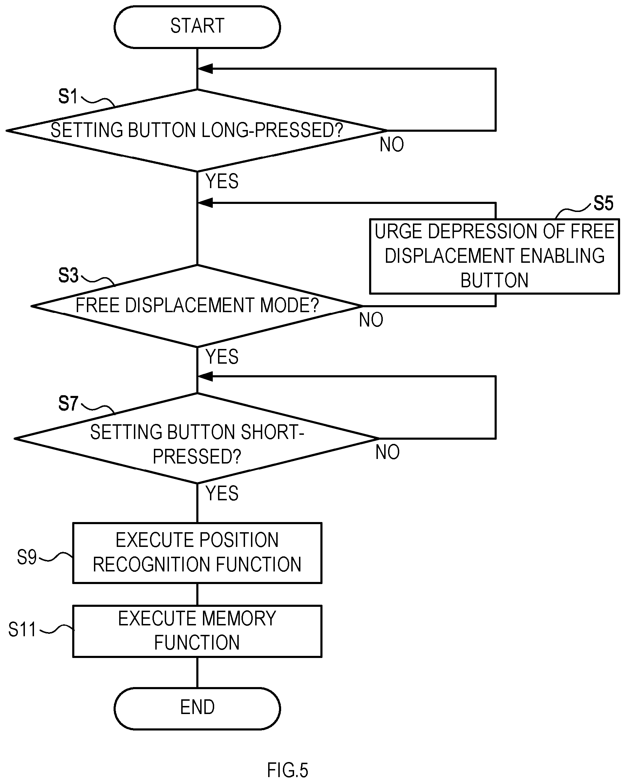

FIG. 5 shows an example control of the control device 5 executed in the immovable point setting mode. The control device 5 determines whether the setting button 17A is depressed for more than a specified time (for example, three seconds) (S1). "(S1)" and the like indicate control step numbers illustrated in FIG.5.

The control device 5, when determining that the setting button 17A is depressed for more than the specified time (S1: YES), determines whether the arm drive device 9 is in the free displacement mode (S3).

The control device 5, when determining that the arm drive device 9 is not in the free displacement mode (S3: NO), urges the user to operate the free displacement enabling button 17B by sound (for example, buzzer) or by a notification device such as a warning light (S5).

The control device 5, if determining that the arm drive device 9 is in the free displacement mode (S3: YES), determines whether the setting button 17A is depressed for less than the specified time (for example, two seconds) (S7).

The control device 5, when determining that the setting button 17A is depressed for less than the specified time (S7: YES), executes the position recognition function (S9), and then executes the memory function (S11).

In other words, in the present embodiment, when the arm drive device 9 is not in the free displacement mode (S3: NO), the position recognition function and the memory function are virtually disabled.

The control device 5, after storing the pivot P₁ as the immovable point, ends the immovable point setting mode and the free displacement mode, and notifies the user that the pivot P₁ is stored as the immovable point.

### <3. Features of surgical robot according to present embodiment>

In the surgical robot 1 according to the present embodiment, the relative positional relationship between the site where the trocar 15 is to be inserted and the distal end position of the surgical instrument 7 during surgery is displayed on the display section 19. This allows the operator to confirm "whether the surgical instrument 7 is moving so that the portion of the surgical instrument 7 corresponding to the incision site is immovable".

In other words, whether the surgical robot 1 "has recognized the site where the trocar 15 is to be inserted as the immovable point", that is, whether surgery by the surgical robot 1 is ready to be performed can be easily and reliably recognized by the operator.

The first display processor 21A uses the position stored by the immovable point setter 11 as the site where the trocar 15 is to be inserted. This allows the operator to easily and reliably recognize whether the immovable point setter 11 stores the incision position as the immovable point.

In the surgical robot 1 according to the present embodiment, the detection result of the drape detector 23 is displayed on the display section 19. This allows the operator to easily and reliably recognize whether surgery by the surgical robot 1 is ready to be performed.

The surgical robot 1 according to the present embodiment recognizes the position of the site where the trocar 15 is to be inserted during surgery, that is, the incision position, and stores the recognized position as the pivot P₁. Thus, in the surgical robot 1, alignment work between the position of the pivot P₁ and the incision site can be easily performed.

The arm drive device 9 can execute the free displacement mode. Thus, in the surgical robot 1, the user can execute the position recognition function and the memory function after aligning the distal end of the surgical instrument 7 with the incision site. Accordingly, alignment work between the position of the pivot P₁ and the incision site can be easily performed.

### (Other Embodiments)

The robot arm 3 according to the aforementioned embodiment is configured by a link mechanism that can change the position of pivot. However, the present disclosure is not limited to the configuration in which the robot arm 3 is configured by a link mechanism that can change the position of pivot. Specifically, for example, the present disclosure may be configured so that the pivot (hereinafter, also referred to as an immovable point) is immovable relative to the robot body.

In the aforementioned embodiment, the control device 5 comprises the second display processor 21B. However, the present disclosure is not limited to the configuration in which the control device 5 comprises the second display processor 21B. The second display processor 21B may be provided in a component other than the control device 5, or the second display processor 21B may be omitted, etc.

In the aforementioned embodiment, if the arm drive device 9 is not in the free displacement mode (S7: NO), the control device 5 disables the position recognition function and the memory function. However, the present disclosure is not limited to the configuration in which the control device 5 disables the recognition function and the memory function if the arm drive device 9 is not in the free displacement mode (S7: NO).

Specifically, for example, the present disclosure may be configured so that, even in a mode other than the free displacement mode, the control device 5 may enable the position recognition function and the memory function. In this case, the control device 5 may use the master-side input operation device to align the distal end of the surgical instrument 7 with the incision position.

The immovable point setter 11 according to the aforementioned embodiment obtains the coordinate representing the distal end position of the surgical instrument 7 from the attitude of the robot arm 3 to recognize the distal end position. However, the present disclosure is not limited to the configuration in which the coordinate representing the distal end position of the surgical instrument 7 is obtained from the attitude of the robot arm 3 to recognize the distal end position. Specifically, for example, the present disclosure may be configured so that the distal end position is recognized with an image analysis technique that uses a 3D camera such as a stereo camera and a depth camera.

In the aforementioned embodiment, the user recognizes the distal end of the surgical instrument 7 or a surgical instrument equivalent, in a state in which the distal end is aligned with the incision position, to recognize the incision position. However, the present disclosure is not limited to the configuration in which the user recognizes the distal end of the surgical instrument 7 or a surgical instrument equivalent, in a state in which the distal end is aligned with the incision position. Specifically, for example, the present disclosure may be configured so that a laser light is applied to the incision position, and the applied position is recognized by an image analysis technique.

In the aforementioned embodiment, when the free displacement enabling button 17B is operated, the free displacement mode is started. However, the present disclosure is not limited to the configuration in which, when the free displacement enabling button 17B is operated, the free displacement mode is started. Specifically, for example, the present disclosure may be configured so that, at the same time as the immovable point setting mode is started, the free displacement mode is automatically started.

Further, the present disclosure is only required to be consistent with the gist of the disclosure described in the aforementioned embodiments, and thus is not limited to the aforementioned embodiments. Accordingly, the present disclosure may be configured in combination of at least two of the aforementioned embodiments, or may be configured without some of the components illustrated in the drawings or described with reference numerals in the aforementioned embodiments.

## Claims

1. A surgical robot (1) for use in endoscopic surgery, the surgical robot (1) comprising:
a robot arm (3) that is configured to hold a surgical instrument (7) used in the endoscopic surgery;
a drive device (9) that is configured to drive the robot arm (3);
a display section (19) for displaying information;
a display processor (21A) that is configured to display a relative positional relationship between a pivot (P₁) and a distal end position (St) of the surgical instrument (7) during surgery on the display section (19), the pivot (P₁) being a position which is an immovable point when the robot arm (3) operates, regardless of a state of the robot arm (3); and
an immovable point setter (11) that is configured to recognize the distal end position (St) (i) by obtaining or calculating a coordinate which indicates the distal end position (St) from an attitude of the robot arm (3), (ii) with an image analysis technique that uses a 3D camera such as a stereo camera and a depth camera, or (iii) by applying a laser light to an incision position and recognizing the applied position by an image analysis technique, and store the recognized distal end position as the pivot (P₁), wherein
the display processor (21A) is configured to show, on the display section (19), (i) a case where the distal end position (St) of the surgical instrument (7) is located inside a body relative to the pivot (P₁), (ii) a case where the distal end position (St) of the surgical instrument (7) is located outside the body relative to the pivot (P₁), and (iii) a state in which an immovable point setting is not yet performed, using image information such as figures, in particular icons.

2. The surgical robot (1) according to claim 1, comprising:
a drape detector (23) that is configured to detect whether a drape (20) that covers the robot arm (3) is attached to the robot arm (3); and
a second display processor (21B) that is configured to display a detection result of the drape detector (23) on the display section (19).

## Patentansprüche

1. Operationsroboter (1) zur Verwendung in endoskopischer Chirurgie, wobei der Operationsroboter (1) enthält:
einen Roboterarm (3), der konfiguriert ist, ein Operationsinstrument (7) zu halten, das bei der endoskopischen Chirurgie verwendet wird;
eine Antriebsvorrichtung (9), die konfiguriert ist, den Roboterarm (3) anzutreiben;
einen Anzeigebereich (19) zum Anzeigen von Informationen;
einen Anzeigeprozessor (21A), der konfiguriert ist, während der Chirurgie auf dem Anzeigeabschnitt (19) ein relatives Positionsverhältnis zwischen einem Drehpunkt (P₁) und einer distalen Endlage (St) des Operationsinstruments (7) anzuzeigen, wobei der Drehpunkt (P₁) eine Position ist, die ein unbeweglicher Punkt ist, wenn der Roboterarm (3) arbeitet, unabhängig von einem Zustand des Roboterarms (3), und
einen unbeweglichen Punkteinsteller (11), der konfiguriert ist, die distale Endposition (St) (i) durch Erhalten oder Berechnen einer Koordinate, die die distale Endposition (St) aus einer Haltung des Roboterarms (3) anzeigt, (ii) mit einer Bildanalysetechnik, die eine 3D-Kamera wie eine Stereokamera und eine Tiefenkamera, verwendet, oder (iii) durch Anwenden eines Laserlichts auf einer Inzisionsposition und Erkennen der angewendeten Position durch ein Bildanalyseverfahren, zu erkennen und die erkannte distalen Endposition als den Drehpunkt (P₁) zu speichern, wobei
der Anzeigeprozessor (21A) konfiguriert ist, auf dem Anzeigebereich (19) (i) einen Fall, in dem sich die distale Endposition (St) des Operationsinstruments (7) innerhalb eines Körpers relativ zum Drehpunkt (P₁) befindet, (ii) einen Fall, in dem sich die distale Endposition (St) des Operationsinstruments (7) außerhalb des Körpers relativ zum Drehpunkt (P₁) befindet, und (iii) einen Zustand zu zeigen, in dem eine unbewegliche Punkteinstellung noch nicht durchgeführt ist, unter Verwendung von Bildinformationen wie Figuren, insbesondere Symbolen.

2. Operationsroboter (1) nach Anspruch 1, enthaltend:
ein Abdeckdetektor (23), der konfiguriert ist, zu detektieren, ob ein Abdeckelement (20), das den Roboterarm (3) abdeckt, an dem Roboterarm (3) befestigt ist; und
einen zweiten Anzeigeprozessor (21B), der konfiguriert ist, ein Detektionsergebnis des Abdeckdetektors (23) auf dem Anzeigebereich (19) anzuzeigen.

## Revendications

1. Un robot chirurgical (1) destiné à être utilisé en chirurgie endoscopique, le robot chirurgical (1) comprenant :
un bras robotique (3) configuré pour tenir un instrument chirurgical (7) utilisé dans la chirurgie endoscopique ;
un dispositif d'entraînement (9) qui est configuré pour entraîner le bras du robot (3) ;
une section d'affichage (19) pour afficher des informations ;
un processeur d'affichage (21A) qui est configuré pour afficher une relation de position relative entre un pivot (P₁) et une position finale distale (St) de l'instrument chirurgical (7) pendant la chirurgie sur la section d'affichage (19), le pivot (P₁) étant une position qui est un point immobile lorsque le bras du robot (3) fonctionne, indépendamment d'un état du bras du robot (3) ; et
un ajusteur de point immobile (11) qui est configuré pour reconnaître la position finale distale (St) (i) en obtenant ou en calculant une coordonnée qui indique la position finale distale (St) à partir d'une attitude du bras du robot (3), (ii) avec une technique d'analyse d'images qui utilise une caméra 3D telle qu'une caméra stéréo et une caméra de profondeur, ou (iii) en appliquant une lumière laser à une position d'incision et en reconnaissant la position appliquée par une technique d'analyse d'image, et en mémorisant la position d'extrémité distale reconnue comme le pivot (P₁), dans lequel
le processeur d'affichage (21A) est configuré pour présenter, sur la section d'affichage (19), (i) un cas où la position finale distale (St) de l'instrument chirurgical (7) est située à l'intérieur d'un corps par rapport au pivot (P₁), (ii) un cas où la position finale distale (St) de l'instrument chirurgical (7) est située à l'extérieur du corps par rapport au pivot (P1), et (iii) un état dans lequel un ajustage de point immobile n'est pas encore effectué, en utilisant des informations d'image telles que des figures, en particulier des icônes.

2. Le robot chirurgical (1) selon la revendication 1, comprenant :
un détecteur de drapé (23) est configuré pour détecter si un drapé (20) qui recouvre le bras du robot (3) est fixé au bras du robot (3) ; et
un second processeur d'affichage (21B) qui est configuré pour afficher un résultat de détection du détecteur de drapé (23) sur la section d'affichage (19).
